# EUROPEAN PATENT APPLICATION

(11) **EP 2 207 031 A1**
(43) Date of publication of application: **14.07.2010**
(21) Application number: 09160509.7
(22) Date of filing: 18.05.2009
(51) Int. Cl.: G01N 33/50, C12N 5/07

(54) **Adjuvant testing for vaccine preparations**

(71) Applicant: Abbott Biologicals B.V., 1381 CP Weesp (NL)
(72) Inventor: Breedveld, Pauline, 1381 CP Weesp (NL); de Gruijl, Tanja, 1081 HV Amsterdam (NL); van de Ven, Rieneke, 1081 HV Amsterdam (NL); Scheper, Riekeld J., 1081 HV Amsterdam (NL)
(74) Representative: Aerts, Robert Jaap

(57) **Abstract**

The present invention belongs to the field of pharmaceutical industry and relates to a method for testing an exogenous adjuvant in an ex vivo skin model, comprising the following steps:
a) providing an ex vivo skin tissue,
b) applying said exogenous adjuvant to be tested to the skin tissue, and
c) determining the immunological status of cells from the skin tissue.

Furthermore, it relates to the use of the method according to the invention for the development of a vaccine preparation and to the use of an exogenous adjuvant selected by the method according to the invention for a vaccine preparation.

Moreover, the present invention relates to the use of an exogenous adjuvant.

## Description

### Field of the invention

The present invention belongs to the field of pharmaceutical industry and relates to a method for testing candidate exogenous adjuvants and to the use of said method for the development or the manufacture of a vaccine preparation. Furthermore, the present invention also relates to the use of an exogenous adjuvant selected by such testing method for a vaccine preparation, and to the use of an exogenous adjuvant, wherein the vaccine preparation contains an antigen and wherein the exogenous adjuvant displays an increased activating ability.

### Description of the background art

The use of vaccines is one of the most cost-effective public health measures, providing a huge impact on lowering disease burden and increasing life expectancy. In general, the basic principle of a vaccine is to induce an immune response that largely resembles the immune response induced by real infections while avoiding unwanted side effects of infectious diseases. A desirable result of the vaccination is immunity, that is, the induction of memory in the adaptive arm of the immune system. Such an immunity is characterized by increased titers of specific antibodies (Ab) directed against the antigen (Ag) being present in the vaccine or vaccine preparation, respectively, and higher frequencies of antigen (Ag)- specific B and T cells with accelerated and increased functional responses in the case of re-encounter of the same Ag.
The magnitude as well as the quality of the adaptive immune response depends on the efficient induction of the innate arm of the immune system. The innate arm of the immune system are host defenses that exist prior to exposure to an antigen and that involve anatomic, physiologic, endocytic and phagocytic, and inflammatory mechanisms. Both, the adaptive immune system as well as the innate immune system, contribute to a specific, strong, and long-lasting immune response, wherefore both systems should be activated. One of the central interfaces between these two systems are dendritic cells (DC), which are considered sentinels for pathogen- or danger-related signals. DCs are activated by such signals and at the same time are the only cells fully equipped to prime naïve T cells. After activation, the naïve T cells proliferate and differentiate into effector T cells, which include the cytokine-secreting T helper cell (T_{H} cell) and the T cytotoxic lymphocyte (T_{C} cell). Some of the cells differentiate in memory cells, being responsible for long and even life-long immunity to many pathogens.

The magnitude and the quality of the immune response can be modulated, e.g. by the nature of the antigen or by an adjuvant.

In order to analyze the effect of an adjuvant and/or antigen on the immune response, there exist in vitro models for adjuvant testing besides preclinical in vivo testing.

In this respect, Seubert et al. ("The Adjuvants Aluminum Hydroxide and MF59 Induce Monocyte and Granulocyte Chemoattractants and Enhance Monocyte Differentiation towards Dendritic Cells", The Journal of Immunology, 2008, 180: pp. 5402-5412) analyze the effect of the adjuvants aluminum hydroxide, MF59 and Lipopolysaccharide (LPS) on human immune cells. For this purpose, a cell based in vitro model was used: Various human cell types being involved in the induction of an immune response were isolated (tissue-resident macrophages (MΦ) and DCs, endothelial and epithelial cells, monocytes) and stimulated in vitro with MF59, alum, or LPS at varying doses and time spans. Afterwards, cytokine and chemokine secretion, phenotypical changes, surface marker expression and migration behaviour have been analyzed.

Furthermore, dos Santos et al. ("Progress on the development of human in vitro dendritic cell based assay for assessment of the sensitizing potential of a compound", Toxicology and Applied Pharmacology, 2009, to be published) describe some dendritic cell-based in vitro models used for the assessment of potential sensitizing nature of compounds.

In WO 2007/108835, the set-up of an artificial immune system (AIS) is described, which can be used for in vitro testing of vaccines, adjuvants, or immunotherapy candidates. In order to come as close as possible to an in vivo situation of the immune system, the international patent application teaches the combination of a modified matrix, mimicking the vaccination site, and a three-dimensional artificial lymphoid tissue, comprising a membrane and a plurality of lymphocytes und leukocytes. According to this international patent application, by seeding epithelial and endothelial cells on the matrix representing the vaccination site, a skin equivalent can be generated. In the AIS, T- and B- cells are present in the artificial lymphoid tissue, however not in the skin equivalent. An analysis of the immune response, also taking into account the effect of T- and, optionally, B-cells in particular on DC, is only intended to be carried out in combination with the downstream three-dimensional artificial lymphoid tissue.

However, despite the above described methods for carrying out adjuvant testing and for assessing the sensitizing nature of candidate compounds, there is still a need for an improved method for testing adjuvants and for useful tools to develop (adjuvanted) vaccine preparations.

### Summary of the invention

The present invention provides the following aspects, subject-matters and preferred embodiments, which respectively taken alone or in combination, contribute to solving the object of the present invention:
(1) A method for testing a candidate exogenous adjuvant in an *ex vivo* skin model, comprising the following steps:
   a) providing an ex vivo skin tissue,
   b) applying said exogenous adjuvant to be tested to the skin tissue, and
   c) determining the immunological status of cells from the skin tissue.
(2) The method according to item (1), wherein in a step d) the immunological status of the cells from the skin tissue is compared to the immunological status of cells from a skin tissue to which no exogenous adjuvant has been applied or to which a reference adjuvant has been applied.
(3) The method according to item (2), wherein a exogenous adjuvant is selected as immunostimulative, if a cell fraction of at least about 30 %, preferably of at least about 40 %, more preferably of at least about 45 % or of at least about 50 %, and most preferably of at least about 60 % has the analyzed activation and/or maturation marker/s expressed.
   It is also possible that a cell fraction of at least about 65 %, 70 % or 75 % has the analyzed activation and/or maturation marker/s expressed.
(4) The method according to any of the preceding items, wherein the method is used for testing an exogenous adjuvant for vaccine preparations.
(5) The method according to any of the preceding items, wherein the skin tissue is cultivated in culture medium for a time period of up to 7 days, preferably up to 5 days, more preferably up to 4 days, even more preferably up to 3 days, and most preferably up to 2 days.
(6) The method according to any of items (2) to (5), wherein in step d) the exogenous reference adjuvant is an adjuvant comprising at least one component selected from the group consisting of cholesterol, saponin, and phospholipid, optionally combined with amphipathic proteins.
(7) The method according to any of the preceding items, wherein the exogenous adjuvant is applied to the skin tissue by invasive or non-invasive methods.
(8) The method according to item (7), wherein the invasive methods comprise injection, preferably intradermally, of the adjuvant, and tattooing of the skin, and the non-invasive methods comprise epicutaneous or transdermal applications such as adjuvant administration via patch or tape-stripping.
(9) The method according to any of the preceding items, wherein the exogenous adjuvant is applied together with an antigen.
(10) The method according to any of the preceding items, wherein the exogenous adjuvant is applied together with a vaccine preparation to be tested.
(11) The method according to any of the preceding items, wherein the candidate exogenous adjuvant is
   a) included in the vaccine preparation, or
   b) added before or after the addition of the vaccine preparation.
      Preferably, the vaccine preparation contains an antigen and excipients, such as buffer components.
(12) The method according to any of the preceding items, wherein the vaccine preparation contains at least one antigen, preferably an inactivated or attenuated virus, more preferably a viral antigen such as a split virus antigen, a subunit virus antigen or a virosome, or the antigen comprises at least one component of a virus or a virus particle, preferably the antigen is an influenza virus particle or an avian influenza virus particle.
(13) The method according to any of the preceding items, wherein the vaccine preparation is an influenza vaccine preparation, preferably a pandemic or an epidemic influenza vaccine preparation, such as an avian influenza vaccine preparation or a seasonal influenza vaccine preparation.
(14) The method according to item (13), wherein the influenza vaccine preparation comprises a hemagglutinin (HA) and/or neuraminidase (NA) antigen.
(15) The method according to any of the preceding claims, wherein the skin tissue is a mammalian skin tissue, preferably a human skin tissue.
(16) The method according to any of the preceding items, wherein the vaccine preparation is added to the skin tissue by invasive or non-invasive means.
(17) The method according to item (16), wherein the invasive methods comprise injection, preferably intradermally, of the adjuvant, and tattooing of the skin, and the non-invasive methods comprise epicutaneous or transdermal applications such as adjuvant administration via patch or tape-stripping.
(18) The method according to any of the preceding items, wherein in step 1 c) the immunological status of the cells from the skin tissue is determined at one time point or at different time points after the removal of the skin tissue from the culture medium, preferably the immunological status is determined subsequently to the removal, and/or 12 hours, 18 hours, 1 day, 2 days, 3 days, 4 days, 5 days, 6 days, 7 days or 8 days after the removal, more preferably subsequently to the removal and 5 days after the removal of the skin tissue from the culture medium.
   In a preferred embodiment, the removal of the skin tissue is carried out after a cultivation time of the skin tissue as described in item (5).
(19) The method according to item (18), wherein the immunological status of the cells from the skin tissue is determined by biochemical methods, wherein said methods comprise analysis of proteins, analysis of nucleic acids, analysis of the ability of the cells from the skin tissue to activate other cells or cell types, and immunohistochemical analysis.
(20) The method according to item (18) or (19), wherein the cells which status is determined are dendritic cells, preferably dermal dendritic cells and monocyte-derived dendritic cells, Langerhans cells, and/or macrophage-like cells.
(21) Use of the method according to any of the preceding claims for the development of, or as a prior step of selecting an exogenous adjuvant for the manufacture of, a vaccine preparation, preferably of an influenza vaccine preparation.
(22) Use of an exogenous adjuvant selected by the testing method according to any of items (1) to (20) as an exogenous adjuvant for a vaccine preparation, preferably for an influenza vaccine preparation, more preferably for a pandemic or an epidemic influenza vaccine preparation according to any of items (12) to (14).
   A pandemic influenza vaccine preparation can, for instance, be an avian or a pig influenza vaccine preparation.
(23) Use of an exogenous adjuvant according to items (21) or (22), wherein the vaccine preparation contains an antigen and wherein the selected exogenous adjuvant displays an increased activating ability, more specifically a dendritic cell (DC) activating and maturating ability.
(24) Use of an exogenous adjuvant according to any of items (21) to (23), wherein the exogenous adjuvant is selected such that said exogenous adjuvant mainly induces a T_{H}1 or T_{H}2 and/or T_{H}17 cell subset, preferably a T_{H}1 or T_{H}2 subset, more preferably both subsets.
   In particular, the induction of both subsets, T_{H}1 and T_{H}2, is preferred in case the exogenous adjuvant is used for a vaccine preparation, preferably an influenza vaccine preparation, and/or in case an anti-influenza response should be elicited.

### Detailed description of the invention

The present invention is now described in more detail by preferred embodiments and examples, which are however presented for illustrative purpose only and shall not be understood as limiting the scope of the present invention in any way.

The present invention provides an efficient and reliable method for testing of adjuvants in a close-to-human in vivo setting, while specifically addressing DC's and/or LC's central role of provoking immune response mechanisms. In general, adjuvants are used in order to boost the immune response when an antigen is considered of having low immunogenicity, or when only small amounts of an antigen are available. Moreover, adjuvants might also be involved in suppressing immune response to a certain antigen. At present, it is assumed that adjuvants lead to prolonged antigen persistence at the site of application, to an enhancement of co-stimulatory signals, to enhanced immunity through induction of dendritic cell maturation and/or to a stimulation of nonspecific proliferation of lymphocytes. DCs are seen as nature's adjuvant and have the potential to recognize foreign antigen, process it into small peptides for presentation onto MHC molecules to the T cell receptor, and to provide the essential costimulatory molecules for activation of naive CD4⁺ and CD8⁺ T cells. However, as the exact mechanism underlying the modulator function of an adjuvant on the immune response is not properly understood, the provision of an efficient and reliable adjuvant to test for its effect on the immune system is highly beneficial. The test method according to the present invention allows for reliably determining the effects of potential exogenous adjuvants on the immune response, more specifically the induction of DC maturation, in living organisms, such as mammals, in particular in human beings. Furthermore, the test method according to the present invention allows for the screening of a high number of candidate adjuvants in a short time without being too expensive. In addition, the ex vivo test method according to the present invention allows for a reduction of candidate adjuvants to be tested in animals during preclinical efficacy studies.

It was surprisingly found that a method for testing an exogenous adjuvant comprising the steps of providing an ex vivo skin tissue, applying said exogenous adjuvant to be tested to the skin tissue and determining the immunological status of the cells from the skin tissue is particularly advantageous and useful for testing the effect of an exogenous adjuvant on the immune response.
In particular it has now surprisingly been found that by using the method according to the present invention, reliable predictions can be made as to the effect of the tested exogenous adjuvant in living organisms such as in mammals, and in particular in human beings. At present the mode of action of adjuvants is complex and not clear. With regard to their function of enhancing the immune response, in addition to the putative effects described above, it is known that the adjuvanticity of an adjuvant is also coupled to its effects on T cell stimulation. However, in skin, the number of resident T cells is very low comparable to the number of T cells being present for instance in lymph nodes, where usually the priming of the T cells by DC takes place. Due to the low number of the skin resident T cells it is not possible to carry out assays using these T cells, for instance assays for assessing the effect of exogenous adjuvants on these T cells. As a consequence, only the effect of the tested exogenous adjuvant on DC and/or LC, which are present in the skin in larger numbers, can and shall be tested, but not the effect of the adjuvant to be tested on T cells. Surprisingly, by applying the model according to the present invention, despite this problem, the inventive method provides a tool for reliably predicting the effects of the adjuvant tested on the immune response of mammals, in particular of human beings. Advantageously, it is however feasible to make reliable exogenous adjuvant testing without the need to set-up a full artificial immune system, hence the addition of a separate, optionally artificial, lymphoid tissue comprising substantial and previously added lymphocytes and leukocytes can be avoided.

Until now, in order to be able to reliably predict the effect of tested adjuvants on the immune system of human beings, animal testing has been necessary. However, animal testing encounter ethical concerns and is in general not well accepted. Moreover, animal skin is, at least to a certain extent, not comparable to human skin, which leads to the situation that the results obtained by animal testing can not reliable be transferred to human beings. This leads to a need for alternative test systems. Possible alternative test systems are for instance in vitro cell based adjuvant assays. However, the effects of adjuvants observed on DC in in vitro test systems do not correlate with the effects of adjuvants observed on DC when using the test method according to the present invention. For instance, in the in vitro model, the use of the adjuvants "Immune Stimulating Complexes" (ISCOM, Matrix M^{®}) or Aluminum hydroxide (AIOH, Alhydrogel) leads to the induction of apoptosis in a high percentage of DC, whereas in the new ex vivo skin model, the DC did not show a high percentage of apoptotic cells but a significant up-regulation of co-stimulatory (CD80/86) and antigen-presenting (HLA-DR/-ABC) molecules. Thus, the method according to the present invention unexpectedly provides an improved test system. While being effective, the present new method can provide reliable and repetitive results in a shorter period of time, yet can be specifically directed towards the organism whose ex vivo skin tissue is chosen and who shall correspondingly be immunologically protected by a suitably chosen adjuvant/antigen combination.
Furthermore, by using the method according to the present invention, the effect of the tested exogenous adjuvant on immune response can be analyzed in the context of intact tissue, including the effects of the adjuvant on the surrounding tissue. This conserved tissue context makes the present model the most close-to-human in vivo test system, as the cross talk between skin residential cells, e.g. epidermal cells and DC and MΦ, is not interrupted, therefore mimicking real life situation. This is in sharp contrast to known in vitro models for testing candidate adjuvants. It has been surprisingly found that by using the method according to the invention it is possible to assess whether a tested exogenous adjuvant may lead to an increased or to a suppressed immune response. The general context underlying the method according to the present invention is to apply a candidate exogenous adjuvant to be tested to a skin tissue and to analyze the cells from the skin tissue that may have been responsible to said adjuvant for instance with respect to specific marker proteins that indicate the activation and/or maturation state of the analyzed cell. Usually, after the application of the exogenous adjuvant to be tested to the skin tissue, the responding cells, usually dendritic cells, Langerhans cells (LC) or MΦ-like cells, migrate from the skin tissue into the culture medium, where these cells can be easily harvested and analyzed e.g. with respect to their protein expression such as cytokine expression or surface protein expression ("surface marker", "marker"). Specifically, the new concept of the present invention allows to make use of the full repertoire of markers or expressed proteins which are indicative for certain immunological status of the cell analyzed. Some examples of surface markers and cytokines, indicative of relevant immune status and immune response, are given below.
This might be particularly useful in the field of vaccine preparations, as in this field there is a great need for effective vaccines or vaccine preparations. Vaccine efficacy largely relies on the capacity of the added adjuvant to activate the immune system. For long term protective effects, the activation of the adaptive arm of the immune system is crucial, as it can generate an immunological memory. DCs bridge the innate and the adaptive immune system, and, consequently, DC activation is vital for the activation of both, cell-mediated and humoral immune response. Therefore, a key characteristic of effective adjuvants, i.e. adjuvants that stimulate the immune response, is the stable activation of DC at the site of vaccination. The method according to the present invention can, in principle, bring together from an anatomic as well as physiologic point of view the model target and the potential vaccination target, thereby improving the value and the reliability of the test results. For example, a stable activation of DCs is **characterized in that** the dendritic cells, usually the migrated DC from the skin tissue, are prevented from a switch from a mature T cell stimulatory CD83⁺ CD1a⁺ phenotype to a CD14⁺ MΦ-like non-stimulatory phenotype by the treatment of the skin tissue, from which the cells originate, with a suitable endogenous adjuvant. The prevention of the switch from a T cell stimulatory phenotype to a non-stimulatory phenotype has the advantage that, by maintaining the stimulatory phenotype, a stable, lasting maturation and/or activation state of the DC can be reached, which, in turn, is important for subsequent immunization in the draining lymph nodes. By using the test method according to the present invention, it can be assessed for instance by analyzing suitable markers being present on the surface of the DC at different time points after application of the adjuvant, e.g. at day 2 and at day 7, whether the tested exogenous adjuvant has prevented such a switch or not.
Furthermore, the present method may also be advantageously used in case where an immune response is intended to be suppressed. This may for instance be the case in field of transplantation medicine, especially with regard to graft rejection. Here, it is desired to avoid an immune response being directed against the graft, thereby avoiding the rejection of the graft. By using the method according to the present invention, an exogenous adjuvant leading to the suppression of an immune response, or even leading to the induction of immunological tolerance, can be detected rapidly and reliably. The term "exogenous adjuvant" as used herein therefore encompasses not only immunogenic adjuvants in classical sense, but also immunosuppressant agents or adjuvant substances therefor.
Moreover, the bioavailability and/or the skin penetration of the adjuvant to be tested is/are also included in the test method, thereby even more mimicking real life situation in addition to the cross talk of skin residential cells.

Additionally, the method according to the present invention can beneficially make use of the interaction between or the combination of, respectively, the antigen used and the adjuvant, as both will influence the strength of the immune response induced in the ex vivo skin model. Therefore, by using the method according to the present invention it is also possible to find out a suitable vaccine preparation/adjuvant-combination, wherein the one or more adjuvant used enhances the immune response in a manner sufficient for vaccination purposes. "Sufficient for vaccination purposes" within the meaning of the present invention means that the DCs being present at the vaccination site exhibit a long-lasting (stable) activation, as described herein. This is all the more advantageously in case vaccine preparations that contain recombinant proteins. These recombinant proteins are devoid of natural occurring danger signals that are able to activate the innate immune system. As a consequence, these vaccines are only poor immunogenic, which is why adjuvants are added to the vaccine formulations. The same typically applies to isolated antigens, such as isolated and purified viral antigens, for example surface antigen proteins. However, adjuvants may also be added to vaccine preparations that contain attenuated or killed pathogens in order to enhance the immune response directed against these pathogens.
Moreover, by applying the method according to the present invention, it is also possible to carry out large scale and/or throughput testings of potential adjuvants, as the present method is suitable for being carried out in a medium-throughput manner.

Thus, an efficient and reliable method for testing adjuvants, providing reliable predictions as to the effect of the tested adjuvant in living organisms, is provided.

In one particular aspect, the present invention relates to a method for testing a candidate exogenous adjuvant in an *ex vivo* skin model, comprising the following steps:
a) providing an ex vivo skin tissue,
b) applying said exogenous adjuvant to be tested to the skin tissue, and
c) determining the immunological status of cells from the skin tissue.

Within the meaning of the present invention, the term "adjuvant" and notably "exogenous adjuvant" relates to any additive that, either alone or in combination with a pharmaceutical preparation, modifies the immune response. The term "candidate" makes clear that by using the model according to the present invention not only already known adjuvants can be screened for their effect on the cells from the skin tissue, but also all kind of compounds that might have an effect on said cells. In particular, by using the model according to the present invention, candidate adjuvants may be selected that lead to a prolonged activation of the cells from the skin tissue as described herein. In case already known adjuvants are tested, by using the method according to the present invention for instance timing and/or vaccination schemes can be assessed and/or tested, as well as proper antigen/exogenous adjuvant combinations or synergistic effects that can potentially arise when combining exogenous adjuvants. Furthermore, said novel method can be used for gathering information about an applied vaccine preparation itself, e.g. in case the vaccine preparation contains a preservative, such as thiomersal, or with regard to local toxicity of the applied vaccine preparation. Moreover, by using the method according to the present invention, possible toxic effects of the exogenous adjuvant to be tested, either alone or in combination with a vaccine preparation. It is known to a person skilled in the art how potentially occurring toxic effects can be detected. For instance, toxic effects can lead to a destruction of normal skin architecture, which may be detected by microscopic inspection.
A pharmaceutical preparation within the meaning of the present invention is a composition that can be used in, or on, the body to prevent, diagnose, alleviate, treat or cure a disease in humans or animals. Preferably, such a pharmaceutical composition is a vaccine preparation or an intermediate product thereof, more preferably an influenza vaccine preparation, such as a seasonal, a pandemic or an epidemic influenza vaccine preparation. Examples of a pandemic or epidemic influenza vaccine preparation are an avian or a pig influenza vaccine preparation.
Modification of the immune response primarily means augmentation, intensification, or broadening the specificity of either or both antibody or cellular immune responses. Modification of the immune response can also mean decreasing or suppressing certain antigen-specific immune responses such as the induction of tolerance. In this regard, the exogenous adjuvant or additive, respectively, can be any compound comprising a modulating effect on the immune response as described above. For example such adjuvant could be of mineral, bacterial, plant, synthetic or host origin or it could be oil in water emulsions.
Whether an additive is an adjuvant within the meaning of the present invention can also be assessed by determining the immunological status of the cells from the skin tissue in step c) as described below.

It is generally agreed that adjuvants are additives that prolong the antigen persistence, enhance the expression of co-stimulatory signals, increase local inflammation and/or to stimulate the non-specific proliferation of lymphocytes. Typical additives exhibiting adjuvanting properties are inorganic adjuvants such as aluminum salts, e.g. aluminum phosphate and aluminum hydroxide (Alhydrogel^{®}), or liposomes. These adjuvants seem to prolong the antigen persistence at the site of injection by precipitating the antigen, thereby slower releasing the antigen from the injection site and enhancing the effective time of exposure to the antigen. Moreover, the size of the antigen is increased, thereby increasing the likelihood of phagocytosis. Further adjuvants typically used are organic adjuvants such as the organic compound squalene, or oil-based adjuvants. Examples of the latter are Freund's incomplete adjuvant containing antigen in aqueous solution, mineral oil, and an emulsifying agent such as mannide monooleate, which disperses the oil into small droplets surrounding the antigen. As a consequence, the antigen is released very slowly from the injection site, which prolongs the time of exposure to the antigen. Freund's complete adjuvant contains heat-killed Mycobacteria as an additional ingredient. The muramyl dipeptide, a component of the mycobacterial cell wall, activates MΦ, which may result in a more potent stimulation of the immune response. Further examples of adjuvants are AlK(SO₄)₂, AiNa(SO₄)₂, AlNH₄(SO₄), silica, Ca₃(PO₄)₂, kaolin, carbon, N-acetyl-muramyl-L-threonyl-D-isoglutamine (thr-DMP), N-acetyl-nornuramyl-L-alanyl-D-isoglutamine (CGP11687, also referred to as nor MDP), Merck Adjuvant 65, ISCOMS^{®}, or synthetic peptide matrixes. By using the method according to the present invention for the screening of already known adjuvants, the effect of this known adjuvant, either alone or in combination with an antigen, on the immunological status of the cells from the skin tissue can be determined. Moreover, the method according to the present invention can also be used for the screening of a suitable modification or optimization of a known adjuvant or for a suitable composition of per se known adjuvants. A modification, optimization, or composition is suitable if it exerts the desired effect on the cells from the skin tissue. Furthermore, by using the method according to the present invention, novel compounds having an effect on the cells from the skin tissue can be identified.

The term "exogenous adjuvant" within the meaning of the present invention denotes a compound or an additive, respectively, that is not present in an individual organism or living cell per se. Such an exogenous compound is opposed to, and excludes an endogenous compound or additive, which is per se present in an individual organism, tissue, or living cell. By using the method according to the present invention, exogenous adjuvants can be tested with respect to their effect on the immune response. In particular, the effect of said adjuvants in combination with a vaccine preparation can be tested. Until now, the use of adjuvants in the infectious field, such as in the vaccine field, has not been very common, which is contrary for instance to the field of dermatology or tumor immunology. In the latter field of activity, the use of adjuvants, in particular the use of endogenous adjuvants such as interleukines or growth factors, is widely spread, for instance in order to reach a proper immune response directed against the tumor antigen.
However, with the rise of recombinant vaccines and recombinant vaccine preparations, there exists a need for a model that allows for the testing of candidate exogenous adjuvants. This is all the more true for the field of influenza vaccine preparations, e.g. as more and more influenza vaccine preparations contain antigens and in particular recombinant antigens that do not possess inherently the capability of inducing a proper, strong immune response.
Examples of endogenous additives or compounds are for instance cytokines, such as interferons, interleukins, colony-stimulating factors, tumor necrosis factors, chemokines, or prostaglandins and leukotrienes. However, this endogenous additives or compounds may be added to the skin tissue or to the culture medium. Cytokines are low-molecular weight regulatory proteins or glycoproteins secreted by white blood cells and various other cells in the body in response to a number of stimuli. These proteins assist in regulating the development of immune effector cells, and some cytokines possess direct effector function on their own. Cytokines bind to specific receptors on the membrane of target cells, triggering signal-transduction pathways that alter gene expression in said target cells. In general, five main groups of cytokines can be distinguished: Interferons (IFNs), interleukins (ILs), colony-stimulating factors (CSFs), tumor necrosis factors (TNFs), and chemokines. IFNs are natural cell-signaling proteins produced by the cells of the immune system of most vertebrates in response to challenges by foreign agents such as viruses, parasites and tumor cells. Examples of IFNs include IFN-γ, mainly secreted by T_{H}1 cells, CD8⁺ cells and natural killer (NK) cells, leading to an activation of macrophages (MΦ), increased expression of major histocompatibility complex (MHC) class I and class II molecules, and to an increased antigen presentation, and IFN-α, mainly secreted by MΦ, leading to the induction of an antiviral state in most nucleated cells, to an increase in MHC class I expression and to an activation of NK cells.
ILs are produced by a wide variety of body cells and regulate large parts of the immune system. The majority of interleukins are synthesized by helper CD4⁺ T lymphocytes, as well as through monocytes, macrophages, and endothelial cells. They promote the development and differentiation of T, B, and hematopoietic cells. Examples of ILs include IL-4, IL-10, IL-6, IL-12p70, IL-23, and IL-8. IL-10 is mainly produced from monocytes T_{H}2 cells, CD8⁺ T cells, MΦ, and B cells, and inhibits the T_{H}1 cytokine production (IFN-γ, TNF-β, IL-2) and promotes antibody production of B cells. IL-4 is mainly produced from T_{H}2-cells, mast cells, MΦ, and CD4⁺ cells, and leads to the proliferation and differentiation of the target cells. Furthermore, IL-4 plays an important role in the generation of allergic responses. IL-6 is mainly produced by MΦ, B cells and T_{H}2 cells and plays a role in antibody secretion and cell differentiation. IL-12 is mainly produced by DC, B cells, T cells and MΦ and induces the differentiation of T cells into cytotoxic T cells together with IL-2, leads to the upregulation of IFN-γ and TNF-α secretion and lowers the expression of IL-10. Chemokines are chemotactical cytokines, inducing directed chemotaxis in responsive cells.
CSFs bind to receptor proteins on the surfaces of hemopoietic stem cells, thereby activating intracellular signaling pathways which, in turn, can cause the cells to proliferate and differentiate into a specific kind of blood cell. One example of a CSF is granulocyte macrophage colony-stimulating factor (GM-CSF), which is mainly secreted by MΦ, T cells, mast cells, endothelial cells, fibroblasts and keratinocytes. GM-CSF stimulates stem cells to produce granulocytes (neutrophils, eosinophils, and basophils) and monocytes. An example for a TNF is TNF-α, which is mainly secreted by MΦ and plays an important role in inflammation.

Furthermore, an exogenous adjuvant within the meaning of the present invention may also denote an additive that is not inherently present in the skin tissue used for carrying out the method according to the present invention.
The human skin consists of two main layers, the outer epidermis and the inner dermis. The epidermis mainly contains cells such as keratinocytes, melanocytes, dendritic cells, Langerhans cells (which are dendritic cells in the epidermis) and Merkels cells, and the inner dermis is mainly composed of the structural components collagen, fibroblasts and extrafibrillar matrix. As a candidate adjuvant to be tested by using the method according to the present invention is an exogenous adjuvant, said adjuvant is preferably a compound that is not produced by the aforementioned cells being present in the skin and/or tissue structures.

In step a) of the method according to the present invention, an ex vivo skin tissue is provided. The skin tissue used in the ex vivo skin model of the present invention can be any skin tissue present in an ex vivo status, either natural or artifical skin tissue. This skin tissue may be subjected to further treating. The term "ex vivo" within the meaning of the present invention denotes that the skin tissue used closely approximates the in vivo situation. Mimicking the in vivo situation means for instance that the cells are present in the context of preferably intact tissue and are allowed for to cross-talk with other skin residential cells. This can for instance be reached by using natural skin tissue that has been taken from a mammal, preferably followed by cultivation of the skin tissue in suitable culture medium, or by using artificial skin tissue.

Natural skin tissue can be obtained from any mammal, such as mouse, rat, rabbit, sheep, horse, cat, dog, pig or from human beings. In a preferred embodiment, the skin tissue is obtained from human beings. The skin tissue can originate from any part of the body of the mammal, in particular from the human being, such as from the abdomen, from the breast or from the foreskin.
Artificial skin tissue can be manufactured by any method that is known to a person skilled in the art, including the mesh scaffolding method and the collagen method. However, the term "artificial skin" within the present invention denotes that in said skin also T and, optionally, B cells, are preferably present. Although for instance T cells are present in mammalian skin, in particular in human skin, only in small amounts, there may also be some cross-talk taking place between skin residential cells, thereby influencing the effect the adjuvant to be tested may have on the immune response. Moreover, it is known to a skilled person that the adjuvanticity of an adjuvant is also coupled to its effects on T cell stimulation. Therefore, in an artificial skin according to the present invention, preferably T cells and, optionally, B cells, are present at least in such a number that cross-talking between the cells being present in the artificial skin is allowed. It is also possible that T and/or B cells are present in the culture medium.
In general, in order to obtain artificial skin tissue, or artificial skin, respectively, donated skin tissue and cells derived from the biopsy and/or cells from cell lines may be used. Donated skin might for instance be of human origin, e.g. from neonatal foreskins removed during circumcision, from volunteers, from surgery samples, from cadavers, etc.. In case fibroblasts are needed, these fibroblasts are separated from the dermal layer of the donated tissue. In the collagen method, usually keratinocytes, in addition to the fibroblasts, are also extracted from the donor tissue. In case the fibroblasts are to be grown on mesh scaffolding, a polymer is created by combining molecules of lactic acid and glycolic, the same elements used to make dissolving sutures. The compound undergoes a chemical reaction resulting in a larger molecule that consists of repeating biodegradable structural units, the so-called polymer scaffold, onto which the obtained fibroblasts are expanded. As the new cells create a layer of dermal skin, the polymer scaffold disintegrates.
In the collagen method, usually a small amount of bovine collagen that can for instance originate from the extensor tendon of animals, such as calves, is extracted, and added to the fibroblasts. The mixture is then dispensed into molds and allowed to come to room temperature. Thereby, the collagen warms, gels, and traps the fibroblasts, thus promoting the growth of new skin cells. Onto this new dermal skin layer, keratinocytes that form the epidermal layer are seeded.
Further artificial skin that can be used is a three-dimensional artificial human skin tissue comprising collagen vitrigel membrane, keratinocytes, DC and fibroblasts. However, as described above, within the meaning of the present invention it is preferred that also T cells and, optionally, B cells, are present in the artificial skin.
Skin tissue within the meaning of the present invention, either artificial or natural, can for instance be a skin explant. An explant within the meaning of the present invention denotes tissue or tissue pieces, respectively, transferred from the organism or from tissue derived from an organism, or transferred from artificial skin tissue, and placed in a culture medium for growth. An explant culture, therefore, refers to the culturing of the tissue or tissue pieces themselves, wherein the cells being present in the tissue or the tissue pieces, respectively, are left in their surrounding extracellular matrix. This has the advantage that by applying this culturing method the in vivo environment is more accurately mimicked, as opposed to in vitro cell cultures. The conserved tissue context makes the present model the most close-to-in vivo. Therefore, the observed behaviour of the cells corresponds to the real in vivo situation to a large extent, for instance by allowing for the cross-talk between the residential cells, i.e. the cells being present in the skin tissue.
Furthermore, it is possible that one skin explant can be derived from one tissue or tissue piece, however, it is also possible that multiple skin explants are derived from the same tissue.
In a further preferred embodiment of the present invention, the skin explant is a biopsy. This skin biopsy can be subjected to any further examination. Any known skin biopsy method can be applied, such as shave biopsy, incisional biopsy, excisional biopsy, curettage biopsy, fine needle aspirate biopsy, punch biopsy, or shave excision. Preferably, the biopsy is a punch biopsy. A punch biopsy is usually carried out with a round shaped knife ranging in size from about 1 mm to about 8 mm. In a preferred embodiment, the punch biopsy has a size, i.e. a diameter, of between about 2 to about 8 mm, preferably of between about 3 to about 7 mm, more preferably of between about 4 to about 6.5 mm, also more preferably of between about 5 to about 6 mm and most preferably of a size of about 6 mm. The punch biopsy preferably has a thickness being in the range of about 1.5 to about 4.5 mm, preferably of about 2 to about 4 mm, more preferably of about 2.5 to about 3.5 mm and most preferably of about 2 to about 3 mm. The obtained punch biopsy is also referred to as skin explant.

According to the method according to the present invention, after step a), a candidate exogenous adjuvant to be tested or identified as described above is added to the skin tissue. The exogenous adjuvant can be added to the skin tissue by invasive or non-invasive methods.
An invasive method is a method that penetrates or breaks the skin and encompasses all invasive methods that are known to a person skilled in the art, for example injection, preferably intradermally, of the exogenous adjuvant, into the skin tissue, and tattooing of the skin.
A non-invasive method is a method that does not create a break in the skin, and encompasses all non-invasive methods that are known to a person skilled in the art, including transdermal or epicutaneous applications such as tape-stripping or application via patch. Tape-stripping means that substances are applied on the skin after removal of the cornified layer from the epidermis by repeated stripping with adhesive tape or film.

In a further preferred embodiment of the method according to the present invention, the exogenous adjuvant to be tested can be applied together with an antigen. With this regard, reference is made to the description below.

As culture medium for the skin tissue, any suitable medium that is known to a person skilled in the art can be used, e.g. the medium DMEM (Dulbecco's Modified Eagle Medium), IMDM (Iscove's Modified Dulbecco's Media), RPMI (Roswell Park Memorial Institute Medium), preferably IMDM or complete RPMI, more preferably IMDM, optionally conditioned with human pooled serum (HPS) or further additives such as antibiotics.

Furthermore, the skin tissue is cultivated in culture medium for a time period suitably responsive for demonstrating an immune response, for example of up to 7 days, preferably of up to 5 days, more preferably of up to 4 days, even more preferably of up to 3 days, and most preferably of up to 2 days, also most preferably of up to 18 hours or of up to 12 hours. During this cultivation period, at different time points, and/or at the end of the respective cultivation period, samples of the skin tissue and/or of the cells from the skin tissue can be withdrawn from the culture for further treatments or for analyzing purposes. Assays that can be carried out are for instance assays described herein, such as assays determining the cytokine- and/or chemokine level, which assays may also allow for to determine the capacity of the DCs to induce a T_{H}1 or T_{H}2 and/or T_{H}17 cell subset, MLR assays, or phenotypic analysis. Moreover, further analysis that can be carried out include incubating the skin tissue or samples thereof or the cells from the skin tissue with cytokines in order to check the stability of the phenotype of the DCs or to increase the longevity of the DCs or to trigger cytokine release by the DCs (e.g. by treatment with CD40L and/or IFNµ or PGE2 (prostaglandin E2)/dexamethasone). In addition, assays analysing the migratory capacity of cells, in particular of DCs from the skin tissue, can be carried out. It is known to a person skilled in the art, how the aforementioned assays can be carried out. Furthermore, it is also possible that during the cultivation period additives or compounds are added to the culture medium, to the skin tissue being present in the culture medium, and/or to the cells from the skin tissue. It is also possible that additives or compounds such as endogenous additives or compounds are added to the skin tissue prior to the cultivation period. After the cultivation period, the skin tissue is usually discarded. However, the skin tissue can also be used for further treatment and/or for further assays, for instance cryosections could be prepared and the skin tissue could be analyzed with regard to the presence or absence of chemokines and/or cytokines, or with regard to proteins that might be expressed in or on the skin tissue. Cryosections can be prepared according to any suitable method that is known to a skilled person. For instance, for the preparation of the cryosections, skin biopsies can be frozen at the start of the culture as negative control, and preferably 1 day and 2 days after the injection of an adjuvant. For freezing biopsies, the biopsies are cultured on rafts supplied with a filter to allow contact with the culture medium in the culture well rather than floating them freely on the culture medium in order to preserve the tissue structure. Skin biopsies are then gently dried with tissue and covered in freezing medium (such as Tissue Tek, a commercially available tissue freezing medium) and are snap-frozen in liquid nitrogen and stored at -80 °C or preferably in liquid nitrogen. 2 µm cryosections are made on a cryotome at - 20 °C, making sure that both, the epidermal and the dermal skin layers, are present within the section.
The culture medium that might contain the cells migrated from the skin tissue into the culture medium upon contact with the exogenous adjuvant to be tested, can be harvested and analyzed, for instance with regard to extracellular markers or cell-independent markers, but also with regard to markers indicating the immunological status of the cells from the skin tissue, subsequent to the removal of the skin tissue from the culture medium, or the culture medium containing the migrated cells can be left for a further time period before being harvested and subjected to analyzing assays, preferably to assays for determining the immunological status of the cells from the skin tissue, including the assays mentioned above. However, also assays analyzing extracellular markers or cell-independent markers can be carried out. For instance, the culture medium can be left in culture for another 12 hours, 18 hours, 1 day, 2 days, 3 days, 4 days, 5 days, 6 days, 7 days or 8 days after the removal of the skin tissue from the culture medium. More preferably, the assays are carried out subsequently after the removal and/or 5 days after the removal of the skin tissue from the culture medium. At each of said time points, or at multiple time points, the assays as described herein can be carried out. It is also possible that the culture medium is stored at conditions known to a person skilled in the art, followed by later analysis.

According to the method according to the present invention, in a step c) the immunological status of the cells from the skin tissue can be determined. Upon the addition of the exogenous adjuvant, either alone or together with an antigen, the immunological status of cells being present in the culture medium and/or in the skin tissue can be determined. Preferably, the immunological status of DC (dermal DC, monocyte derived DC), Langerhans cells (LC), and/or macrophage-like cells can be determined, preferably the status of LC and/or dermal DC is determined.

As already described above, the immunological status of the cells from the skin tissue can be determined at one time point or at different time points after the removal of the skin tissue from the culture medium, preferably the immunological status is determined subsequently to the removal, and/or 12 hours, 18 hours, 1 day, 2 days, 3 days, 4 days, 5 days, 6 days, 7 days or 8 days after the removal, more preferably subsequently to the removal and/or 5days after the removal of the skin tissue from the culture medium. The immunological status of a cell denotes, on a first level, the status of the cell itself, e.g. the activation and/or maturation status of the cell, e.g. displayed by a certain cytokine expression pattern or by the expression of specific cell surface molecules, and, on a second level, the effect said cell might have on other cells. These effects might either be of activating nature, or of suppressing nature, for instance, said cells might activate the immune response or induce immunological tolerance.
In general, the effect said cell has on other cells or cell types is coupled to the activation/maturation status of said cell. For instance, upon activation, DCs up-regulate the expression of the co-stimulatory surface markers CD80/CD86, thereby providing co-stimulatory signals necessary for T cell activation and survival. CD86 is the ligand for two proteins at the cell surface of T cells, CD28 antigen and CTLA-4 (cytotoxic T-lymphocyte-associated protein 4), and is also known as B7.2. Its principal mode of action is by binding to CD28. Along with CD80, these molecules provide the necessary stimuli to prime T cells against antigens presented by antigen-presenting cells.
It is known to a person skilled in the art how the activation/maturation status of a cell can be determined. Usually, upon activation and/or maturation, a cell changes its phenotype, including its morphology, expression pattern of certain molecules being present in the cell or on the cell surface, and/or the secretion pattern of certain molecules. These changes can be observed by different analytical methods that are known to skilled persons, e.g. by biochemical methods. These methods comprise, for instance, the analysis of proteins, such as the proteins expressed by the cells and, optionally, released in the culture medium, for instance by flow cytometric analysis (fluorescent activated cell sorting, FACS), analysis of nucleic acids, for instance by mRNA analysis (quantitative PCR, qPCR: analysis of cytokine expression), analysis of the ability of the cells from the skin tissue to activate other cells or cell types (cytotoxic T lymphocyte (CTL) assay, mixed-lymphocyte reaction, MLR), and/or immunohistochemical analysis such as the preparation of cryosections, optionally followed by further analysis such as staining of certain proteins and/or cell structures.
According to the present invention, the immunological status of the cells from the skin tissue is determined, preferably from DC, such as dermal DC or monocyte-derived DC, LC, and/or macrophage-like cells.

With regard to dendritic cells, their immunological status can for instance be assessed by analyzing the up-regulation of the expression of co-stimulatory molecules ("activation marker") such as CD80, CD86, and/or CD40 on the cell surface, or by the cytokine expression pattern. Upon activation, DCs up-regulate this co-stimulatory molecules that act as co-receptors in T cell activation, thereby greatly enhancing their ability to activate T cells. They also up-regulate chemokine receptor (CCR) 7, a chemotactic receptor that induces the dendritic cell to migrate from the site of infection and to travel through the blood stream to the spleen or through the lymphatic system to a lymph node. Here, they act as antigen-presenting cells (APCs): They activate helper T cells and killer T cells as well as B cells by presenting them antigens derived from the pathogen, alongside non-antigen specific co-stimulatory signals. Furthermore, in order to determine the immunological status of the cells from the skin tissue, also the cytokine expression pattern of the cells can be analyzed according to standard protocols. For this purpose, the cytokines being present in the respective cells or in the culture medium might be analyzed, e.g. by ELISA (enzyme-linked immunosorbent assay), or by CBA (cytometric bead array), or by analysis of nucleic acids, or by analysing immunohistochemical analysis. All the assays mentioned in the disclosure can, e.g, be carried out according to manufacturer's instructions providing a suitable assay. However, these instructions might also be modified, if necessary and/or if desired. The respective modifications that might be carried out are known to a skilled person. As it is known to a person skilled in the art, different cytokines have different regulatory functions within the immune system. For instance, the cytokine environment in which antigen-primed T_{H} cells differentiate determines the subset of T_{H} cells that develops. In particular, IL-4 is essential for the development of a T_{H}2 response, and IFN-γ, IL-12, and IL-18 are important in the development of T_{H}1 cells, IL-23, IL-6, and TGFβ are important in the development of Tₕ17 cells, and, possibly, IL-10 and TGFβ in the development of T_{reg} cells.
Thus, the observed cytokine release profile provides information on the type of immune response (T_{H}1 and/or T_{H}2) that might be generated by the analyzed cell types after the application of an exogenous adjuvant to be tested. This might be particularly useful in case candidate exogenous adjuvants are screened for an exogenous adjuvant primarily inducing either T_{H}1 response or T_{H}2 response. Which kind of T cell response should be induced by the exogenous adjuvant to be tested mainly depends on the kind of immune response one wants to induce: As it is known to skilled persons, the T_{H}1 response produces a cytokine profile that supports inflammation and activates mainly certain T cells and MΦ, whereas the T_{H}2 response activates mainly B cells and immune responses that depend upon antibodies.
In general, all cytokines one is interested in might be analyzed, preferably, the cytokines IL-15, IL-23, IL-10, IL-12p70, IL-6, IL-8, IL-1β, and TNF-α are analyzed. Furthermore, the cytokine release profile, as well as the surface markers being expressed on the surface of the analyzed cell, informs about the activation/maturation status of said cell (see below). Moreover, surface markers (i.e. molecules that are expressed at the cell surface) known to be specifically expressed by a certain cell type can be analyzed, thereby being able to assess the type of cell that is present in the cell culture and/or in the skin tissue. Furthermore, it is known to a person skilled in the art which marker or which pattern of markers is specifically expressed and/or up- or down-regulated by a certain cell type. For instance, CD14 is a marker being expressed and/or up-regulated by immature DC and by MΦ, CD1a is a marker being expressed and/or up-regulated by cutaneous DC (cDC; LC and dermal DC, DDC), CD83, CD80, and CD86 are expressed and/or up-regulated by activated DC, wherein CD80 and CD86 are co-stimulatory markers being involved in T cell activation and proliferation, and Human Leucocyte Antigen (HLA)-DR/HLA-ABC, also known as antigen-presentating markers or Major Histocompatibility Complex, MHC, is also expressed and/or up-regulated by activated DC. Further cell surface molecules or markers, respectively, that may be analyzed include DC-SIGN, Langerin, CD141/BDCA-3, CD11c, CD64, CXCR4, CCR7, CD205 (DEC-205), CD206 (MMR), CD36, Factor Xllla, CCR5, CCR6, ATPase, and E-cadherin. Table I (J Leukoc Biol. 2008, 84(1):143-51; Santegoets et al.) shows an overview of the markers that are specific for a certain cell type, namely for DDC and/or LC, as stated in literature and as determined by high density microarray analysis. Table II (J Leukoc Biol. 2008, 84(1):143-51; Santegoets et al.) shows relative expression levels of cytokines and cytokine receptors (table II, upper part) and relative expression levels of chemokines and chemokine receptors (table II, lower part), as assessed by transcriptional profiling of human skin-resident Langerhans cells and CD1a⁺ cells. As can be seen, differential activation states suggest distinct functions.

In addition to the markers shown in table I, CD1d is expressed by DDC, EpCAM (epithelial cell adhesion molecule) by LC, and Sulfo LacNac (SLAN) by inflammatory DC. However, it is noted that present table I presents only illustrative examples of possible markers without being complete, and further markers may be regarded as indicative for a certain cell type.

### Table I:

**TABLE I. Differential Characteristics of CD1a⁺ DDC and LC**

| | Literature | | mRNA expression profile | |
|---|---|---|---|---|
| Gene | DDC*^{a}* | LC*^{a}* | CD1a⁺ DDC*^{b}* | LC*^{b}* |
| CD1a | + | + | + + | + + + |
| CD205, DEC-205 | + | + | + | ++ |
| CD206. MMR | + | - | + + | - |
| CD207. Langerin | - | + | ++ | - |
| CD209, DC-SICN | + | - | + | - |
| CD36 | + | - | + - | |
| Factor XIIIa | + | - | + + | - |
| CCR5 | + | - | ++ | - |
| CCR6 | - | + | - | + |
| ATP_{asc} | - | + | - | + |
| E-cadherin | - | + | + + | + + + |

| | | | | |
|---|---|---|---|---|
| "Expression described in literature; reviewed by Valladeau and Saeland [31] and Larregina and Falo [32]. Values given: -, Absent, +. present. "Determined by high-density microarray analysis. Signal intensity levels: -. <200; +. 200-500; + + , 500-5000; +++, >5000. | | | | |

**Table II:**

| Relative Expression Levels of Cytokines and Cytokine Receptors | | | |
|---|---|---|---|
| Gene | CDla⁺ DDC^{a} | LC^{c} | UniGene ID |
| IL-1α. | ++ | ++ | Hs. 1722 |
| IL-1β | +++ | +++ | Hs. 126256 |
| IL-6 | ++ | - | Hs. 512234 |
| IL-8 | +++ | +++ | Hs. 624 |
| IL-10 | ++ | - | Hs. 193717 |
| IL-15 | ++ | - | Hs. 163132 |
| IL-16 | + | - | Hs. 1703359 |
| IL--18 | + | + | Hs. 8::!()?7 |
| IFN-β1 | + | - | Hs. 93177 |
| IL-23p19 | ++ | + | Hs. 98309 |
| IL-1R1 | +++ | +++ | Hs. 82112 |
| IL-1R2 | ++ | ++ | Hs. 25333 |
| IL-1R3 | + | ++ | Hs.143527 |
| IL-1RN | ++ | - | Hs. 81134 |
| IL-4R | ++ | + | Hs. 75545 |
| IL-7R | ++ | - | Hs. 362807 |
| IL-10Rα | ++ | ++ | Hs. 327 |
| IL-13Rα1 | ++ | ++ | Hs. 285115 |
| IL-18BP | - | ++ | Hs. 325978 |
| IL-18R1 | + | + | Hs. 159301 |
| IL-13Rβ | + | - | Hs. 158315 |
| IFN-γR1 | ++ | ++ | Hs. 180866 |
| IFN-γR2 | ++ | ++ | Hs. 409200 |
| CM-CSFR | + | - | Hs. 520937 |

| Relative Expression Levels of Chemokines and Chemokine Receptors | | | |
|---|---|---|---|
| Gene | CDIa⁺ DDC^{a} | LC^{a} | UniGene ID |
| CCL3. MIP-1α | +++ | - | Hs. 73817 |
| CCL4. MIP-1β | +++ | ++ | Hs. 75703 |
| CCL19. MIP-3β | + | - | Hs. 50002 |
| CCL20. MF-3α | +++ | ++ | Hs. 75498 |
| CCL22. MDC | +++ | +++ | Hs. 97203 |
| CXCL1. Gro-α | + + | - | Hs. 789 |
| CXCL12. SDF-α | + | - | Hs. 436042 |
| CXCL2. MIP-2α | +++ | - | Hs. 75765 |
| CXCL3. MIP-2β | +++ | - | Hs. 89690 |
| CCR5 | ++ | - | Hs. 511796 |
| CCR6 | - | + | Hs. 46468 |
| CCR7 | +++ | - | Hs. 1652 |
| CXCR4 | +++ | ++ | Hs. 421986 |
| C3aR1 | ++ | - | Hs. 155935 |
| C5aR1 | ++ | - | Hs. 2161 |
| FPRL2 | ++ | + | Hs. 445466 |

| | | | |
|---|---|---|---|
| ^{α} Signal intensity levels: - <200; +, 200-500; ++, 500-5000; +++, >5000. IL-IRN, IL-IR antagonist; IL-18BP. IL-18 binding protein. ^{a} Signal intensity Levels: -, <200; +, 200-500; ++, 500-5000; +++, >5000. MDC, Monocyte-derived chemokine; Gro-α, growth-relsted oncogene-α; SDF-α stromal cell-derived factor α; FPRL2, formylpeptide receptor-like 2. | | | |

As mentioned above, cells might have an activating or suppressing effect on other cells or cell types ("responder cells"), respectively, by direct and/or indirect interaction with the responder cells. Direct interaction involves a direct physical contact between said cell and the responder cell, whereas an indirect interaction comprises the communication between said cell and the responder cell e.g. via signalling molecules, such as cytokines, or an indirect binding of said cell with a responder cell via a third component, such as an antigen. The term "activating effect" with in the meaning of the present invention denotes that the responder cells, e.g. the T cells, are activated.
In general, it is known to a person skilled in the art which activation/maturation status of the DC leads to the activation (see above) or to the suppression of the responder cells, thereby leading to the induction of immunological tolerance. Within the meaning of the present invention, the term "tolerance" denotes a state of immunological unresponsiveness to particular antigens. As it is known to a person skilled in the art, e.g the expression of the surface markers B7-H1 and/or B7-H4 and/or the cytokines IL-10 and/or TGFβ might be involved in inducing tolerance. Therefore, in case the cells being analyzed express said molecules, this might be evidence that tolerance is induced.
Furthermore, as mentioned above, by using the method according to the present invention it is possible to screen for exogenous adjuvants skewing the immune response towards a specific T helper (T_{H})-profile. With this respect, the method according to the present invention is particularly useful, as the results obtained may be transferred to the human in vivo situation quite reliably, which is contrary to results that are e.g. obtained from animal testing:

For instance with regard to mice models, the induction of the specific (T_{H})-profile varies from strain to strain and, thus, is not very predictably with regard to the human in vivo situation. As a further consequence, the results obtained in mice models are not comparable with the human in vivo situation. As described above, a first hint which T_{H-}profile is induced might be the cytokine pattern expressed by the cells from the skin tissue, preferably from the DC. A further possibility is to co-culture DC that have been migrated from skin tissue to which the exogenous adjuvant has been applied to with allogenic T cells, followed by an analysis of the gene expression profile of the T cells. It is known to a person skilled in the art which kind of gene expression profile is indicative of a T_{H}1, T_{H}2, T_{reg} (regulatory T cell) or T_{H}17 T cell. For example, the expression of T-Bet, an transcription factor and key regulator in determining subset-commitment and cross-regulation, is indicative of the induction of T_{H}1 T cells, the expression of GATA-3, a transcription factor and key regulator in determining subset-commitment and cross-regulation as well, indicates to the skewing towards a T_{H}2 profile, the expression of FOXP3 (forkhead box P3) to the skewing towards a T_{reg} profile, and the expression of RORt to the skewing towards the T_{H}17 profile. The T_{H}1 subset is responsible for many cell-mediated functions, such as delayed-type hypersensitivity and activation of T_{C} cells, and for the production of opsonization-promoting IgG-antibodies. Furthermore, this subset is involved in the promotion of excessive inflammation and tissue injury. The T_{H}2 subset inter alia stimulates eosinophil activation and differentiation and also supports allergic reactions. In case the method according to the present invention is used for screening exogenous adjuvants for vaccines, preferably for influenza vaccines, the exogenous adjuvant is selected such that it induces both subsets, the T_{H}1 subset as well as the T_{H}2 subset.

In a further preferred embodiment according to the present invention, in a step d) the immunological status of the cells from the skin tissue is compared to the immunological status of cells from a skin tissue to which no exogenous adjuvant has been applied or to which an exogenous reference adjuvant has been applied.
A "reference adjuvant" within the meaning of the present invention is an adjuvant that is commonly known to induce a certain, preferably a desired, high activation/maturation status of cells to which it is applied. As an alternative to a reference adjuvant, also endogenous modulators of the immune response may be used, such as chemokines like IL-4 and/or GM-CSF, which are known to induce a strong activation/maturation of the cells from the skin tissue, in particular of the DC.
Whether the activation/maturation status of an analyzed cell is high or not can for instance be determined, and subsequently used as reference, by carrying out any suitable assay that is known to a skilled person, for instance by analysis of the expression of suitable activation/maturation markers, e.g. as described above, expressed by cells being present in the culture medium of the skin tissue cultures, e.g. expressed by DC (dermal DC, monocyte-derived DC), LC, or MΦ-like cells.

Furthermore, the expression of pro-inflammatory chemokines and/or cytokines (see above) by said cells can be analyzed. The analysis of the expression of said markers and chemokines/cytokines can be carried out by any method that is known to a person skilled in the art, for instance by the methods as described above such as flowcytometric analysis, ELISA, CBA, and/or mRNA analysis. As described above, the assays, i.e. the determination of the immunological status of the cells, are/is carried out for instance at one time point or at different time points after the removal of the skin tissue from the culture medium, preferably the immunological status is determined subsequent to the removal, and/or 12 hours, 18 hours, 1 day, 2 days, 3 days, 4 days, 5 days, 6 days, 7 days or 8 days after the removal, more preferably subsequently to the removal and/or 7 days after the removal of the skin tissue from the culture medium. Also preferred, the immunological status of said cells may be determined at one or more optional time points after the removal of the skin tissue from the culture medium. It is also possible to determine the immunological status of cells from the skin tissue in case the skin tissue has not (or not yet) been removed from the culture medium.

Depending on the fraction of cells expressing surface markers being known as indicating activation and/or maturation, a person skilled in the art is able to determine the activation and/or maturation status of the cells. In general, in case that after addition of a reference adjuvant a cell fraction of at least about 30 %, preferably of at least about 40 %, more preferably of at least about 45 % or of at least about 50 %, and most preferably of at least about 60 % has the analyzed activation and/or maturation marker/s expressed, and this activation/maturation status is maintained over a period of at least 7 days (see above), this reference adjuvant can be regarded as an adjuvant inducing a high activation and/or maturation status and, therefore, is immunostimulative. In particular, in case of DC from the skin tissue are analyzed, and in case these DC are prevented from a switch from a mature T cell stimulatory CD83⁺ CD1a⁺ phenotype to a CD14⁺ MΦ-like non-stimulatory phenotype, the reference adjuvant can be regarded as an adjuvant inducing a high activation and/or maturation status. However, whether a candidate exogenous adjuvant is an adjuvant inducing a high activation and/or maturation status, can also be assessed without comparing the immunological status of the cells from the skin tissue to which a candidate adjuvant has been applied with the immunological status of cells from skin tissue to which a reference adjuvant has been applied: In case the cells from the skin tissue to which a candidate exogenous adjuvant has been applied exhibit a cell fraction as indicated above, having the activation and/or maturation marker/s expressed, and in case the analyzed cells are further prevented from a switch to a non-stimulatory phenotype, this candidate exogenous adjuvant is an adjuvant inducing a high activation and/or maturation status.
Furthermore, in case the expression pattern of chemokines and/or cytokines of cells from a skin tissue to which an candidate adjuvant has been applied is analyzed, the candidate adjuvant can be regarded as an adjuvant inducing a high activation and/or maturation status in case the chemokine and/or cytokine expression is comparable to the respective chemokine and/or cytokine expression of cells from skin tissue to which a reference adjuvant has been applied. With this regard, adjuvants comprising at least one component selected from the group consisting of cholesterol, saponin, and phospholipid, optionally combined with amphipathic proteins, can be used as reference adjuvants. Examples of suitable reference adjuvants comprise Immune Stimulating Complexes (ISCOMs), such as Matrix M^{®}. Accordingly, in case the cells from a skin tissue to which an exogenous adjuvant to be tested has been applied exhibit an immunological status being comparable or being higher compared to the immunological status of cells from skin tissue to which a reference adjuvant has been applied, these cells exhibit a high activation/maturation status. In this case, the exogenous adjuvant tested is particularly suitable e.g. for being used in combination with pharmaceutical compositions such as vaccine preparations.
Furthermore, the immunological status of the cells from the skin tissue being present in the culture medium can be compared to the immunological status of cells from a skin tissue to which no exogenous adjuvant has been applied. In this case, the determined immunological status of the cells from the skin tissue to which no exogenous adjuvant has been added can be regarded as negative control. As described above, in case the cells from the skin tissue to which an exogenous adjuvant to be tested has been applied exhibit an immunological status displaying a high maturation/activation status as described above, the exogenous adjuvant tested is suitable for being used in combination with pharmaceutical compositions such as vaccine preparations.

In a further preferred embodiment, the method according to the present invention is used for testing an exogenous adjuvant for vaccine preparations.

Vaccine preparations, or vaccines, respectively, within the meaning of the present invention are preparations that are used to deliver protection against pathogenic threads and can prevent seasonal endemic or pandemic spreads of pathogens. In case of an pandemic or epidemic spread of influenza, a significant gap between influenza vaccine manufacturing capacities and vaccine demands are expected on a global scale. A lower amount of antigen per dose can be increased by the use of suitable adjuvants, therefore increasing the number of providable vaccine doses. Vaccine efficiency largely relies on the capacity of the added adjuvant to activate the immune system. For long term protective effects, the activation of the adaptive arm if the immune system is crucial, as it can generate an immunological memory. In this respect, DC bridge the innate and the adaptive immune system and DC activation is vital for the activation of both cell-mediated and humoral immune responses. Hence a key characteristic of effective immuno-adjuvants should be the stable activation of DC at the vaccination site. By using the method according to the present invention, a suitable exogenous adjuvant for vaccine preparations being able to induce a high activation/maturation status of cells being involved in immune response, preferably of DC, can be identified or detected.

Vaccine preparations within the meaning of the present invention are preparations that establish or improve immune response directed against one or more antigen/s being present in the vaccine preparation.
The at least one antigen within the meaning of the present invention being contained in the vaccine preparation is any chemical or biological material or compound which leads to the generation of antibodies and of an immune response. Preferably, the antigen is an inactivated or attenuated virus, and more preferably the antigen is a viral antigen. Examples of these viral antigens are for instance virus particles such as partially disrupted virus particles such as split virus antigens, purified envelope antigens such as subunit virus antigens or virosomes. A virosome is a unilamellar phospholipid bilayer vesicle with a suitable mean diameter, for example in the range of from about 70 nm to about 150 nm. Essentially, virosomes represent reconstituted empty virus envelopes, devoid of the nucleocapsid including the genetic material of the source virus. Virosomes are not able to replicate but are pure fusion-active vesicles that contain functional viral envelope glycoproteins such as influenza virus hemagglutinin (HA) and neuraminidase (NA) intercalated in the phospholipid bilayer membrane. Also preferred, the vaccine preparation comprises at least one component of a virus or a virus particle, preferably the antigen is an influenza virus particle, more preferably a pandemic influenza virus particle, such as an avian influenza virus particle.

The method according to the present invention is especially useful in the field of testing exogenous adjuvants for influenza vaccine preparations, and beneficially for pandemic or epidemic seasonal influenza vaccine preparations or avian influenza vaccine preparations. As described above, in case of a pandemic influenza spread, there is a high demand on vaccine preparations on a local or global scale. Preferably, these vaccines should be available within a short time. As it usually takes at least several months to generate new vaccine doses, the number of providable doses can also be increased by the use of suitable adjuvants activating and/or maturating immune cells, preferably DC.
Therefore, in a preferred embodiment, the vaccine preparation for which the exogenous adjuvant is tested is an influenza vaccine preparation, more preferably a pandemic or an epidemic influenza vaccine preparation such as a seasonal influenza vaccine preparation, or an avian influenza vaccine preparation. The influenza vaccine preparations can be based on any suitable influenza strain(s). Influenza vaccine preparations typically include antigens from at least one strain of influenza A, B and C virus, preferably from at least one strain of influenza A or B. In a preferred embodiment according to the present invention, the influenza vaccine preparations comprises a HA and/or NA antigen. The recommended strains for vaccine preparations can change from season to season. It may also be possible that the vaccine preparation is based on more than one suitable influenza strain. For instance, the influenza vaccine preparation can include two influenza A strains and one influenza B strain. It may further be possible that the vaccine preparation can not only be a mono-, but also a bi- , tri- or multivalent preparation.
Furthermore, it is also possible that the influenza vaccine preparation only comprises parts of the above mentioned antigens, such as HA and/or NA. Hemagglutinin can for instance be found on the surface of the influenza viruses. It is an antigenic glycoprotein that is responsible for binding the virus to the cell that is being infected. To date, at least 16 different influenza HA antigens are known. These subtypes are named H1 through H16. NA is an enzyme which cleaves the glycosidic linkages of neuraminic acid. To date, at least nine subtypes of influenza neuraminidase are known. These subtypes can be found, for instance, in databases that are known to persons skilled in the art, such as the PubMed database (e.g. http://www.ncbi.nlm.nih.gov/, http://www.ncbi.nlm.nih.gov/genomes/FLU/FLU.html, and htt://www.ncbi.nlm.nih.gov/nuccore/145284465?ordinalpos=1&itoo=EntrezSystem2.PEtre z.Senquence.Sequence ResultsPanel.Sequence RVDocSum). In a preferred embodiment, the vaccine preparation contains any of these HA and/or NA proteins, either alone or in combination with each other. It is also possible that the vaccine preparation contains only parts of these proteins. Preferably, the vaccine preparation contains the complete protein or a part of the proteins H1, H2, H3, H5, H6, H7, N1, N2, N3 or N7, either alone or in combination, preferably the protein or parts of the protein H5.

An antigen within the meaning of the present invention can also be a polynucleotide encoding the antigen as described aboce. This polynucleotide can be present in the vaccine preparation. Furthermore, the polynucleotide might also be inserted in a vector, preferably an expression vector.

The term "polynucleotide" as used herein is to be understood as meaning a double-stranded or single-stranded nucleic acid molecule, e.g. a DNA, cDNA, genomic DNA, RNA and/or mRNA molecule or the like. The nucleic acid molecule can be present either as the coding strand or as the complementary strand. The polynucleotide may be of a natural source or produced by gene technological or chemical processes and synthesis methods or may have been derived there from.

In a further preferred embodiment of the present invention, the vaccine preparation is added to the skin tissue by invasive or non-invasive methods.
An invasive method is a method that penetrates or breaks the skin and encompasses all invasive methods that are known to a person skilled in the art, for example injection, preferably intradermally, of the exogenous adjuvant, into the skin tissue, and tattooing of the skin. A non-invasive method is a method that does not create a break in the skin, and encompasses all non-invasive methods that are known to a person skilled in the art, for example epicutaneous or transdermal applications such as adjuvant administration via patch or tape-stripping.
The vaccine preparation can be added to the skin tissue prior to the onset of the tissue culture, or with or after the onset of the tissue culture. Preferably, the vaccine preparation is added to the skin tissue with the onset of the tissue culture.

In a further preferred embodiment of the present invention, the exogenous adjuvant to be tested is applied together with a vaccine preparation to be tested.
Thereby, the exogenous adjuvant may either be included in the vaccine preparation, or added before or after the addition of the vaccine preparation. In case the exogenous adjuvant is added prior to the addition of the vaccine preparation, it may be added 5 days, preferably 4 days, more preferably 3 days, and even more preferably 2 days prior to the addition of the vaccine preparation. It is also possible that the exogenous adjuvant is added to the skin tissue after the application of the vaccine preparation in order to provide a post- vaccination booster. In this case, the extrogenous adjuvant is added to the skin tissue within a time period of about 4 h to about 48 h, more preferably of about 4 h to 24 h. Preferably the exogenous adjuvant is included in the vaccine preparation.

Furthermore, the present invention relates to the use of the method according to the present invention for the development of, or as a prior step of selecting an exogenous adjuvant for the manufacture of, a vaccine preparation, preferably of an influenza vaccine preparation, and more preferably of a pandemic influenza vaccine preparation, even more preferable of an avian vaccine preparation.

Furthermore, the present invention is also directed to the use of an adjuvant selected by the testing method according to the present invention as an exogenous adjuvant for a vaccine preparation, preferably for an influenza vaccine preparation, more preferably for a pandemic or epidemic influenza vaccine preparation such as a seasonal influenza vaccine preparation or an avian influenza vaccine preparation as described in the present invention. In particular, said exogenous adjuvant is used for a vaccine preparation, wherein the vaccine preparation contains an antigen as described above and wherein the exogenous adjuvant displays an increased activating ability, i.e. the exogenous adjuvant is able to induce an increased activation status, preferably a high activation status, of the analyzed cells as described above. With regard to the determination of the activation status of analyzed cells, reference is made to the description above, describing the determining of the immunological status of cells. By the method according to the present invention, new exogenous adjuvants different from previously known adjuvants can be identified and thereby selected to be included in a vaccine preparation. Moreover, improved and/or optimized modifications of already known exogenous adjuvants can be identified and selected.
Furthermore, the present invention is also directed to the use of an exogenous adjuvant selected according to the method of the present invention, wherein the exogenous adjuvant is selected such that said exogenous adjuvant mainly induces a T_{H}1 or T_{H}2 T cell subset, or both subsets. As described above, in particular in case the exogenous adjuvant is intended to be used in combination with a vaccine preparation, the exogenous adjuvant is selected such that it induces a T_{H}1 or T_{H}2 and/or T_{H}17 cell subset, preferably a T_{H}1 or T_{H}2 subset, more preferably both subsets.

The following drawings and examples illustrate the present invention in more detail, which are however presented for illustrative purpose only and shall not be understood as limiting the scope of the present invention in any way.

### Description of the figures

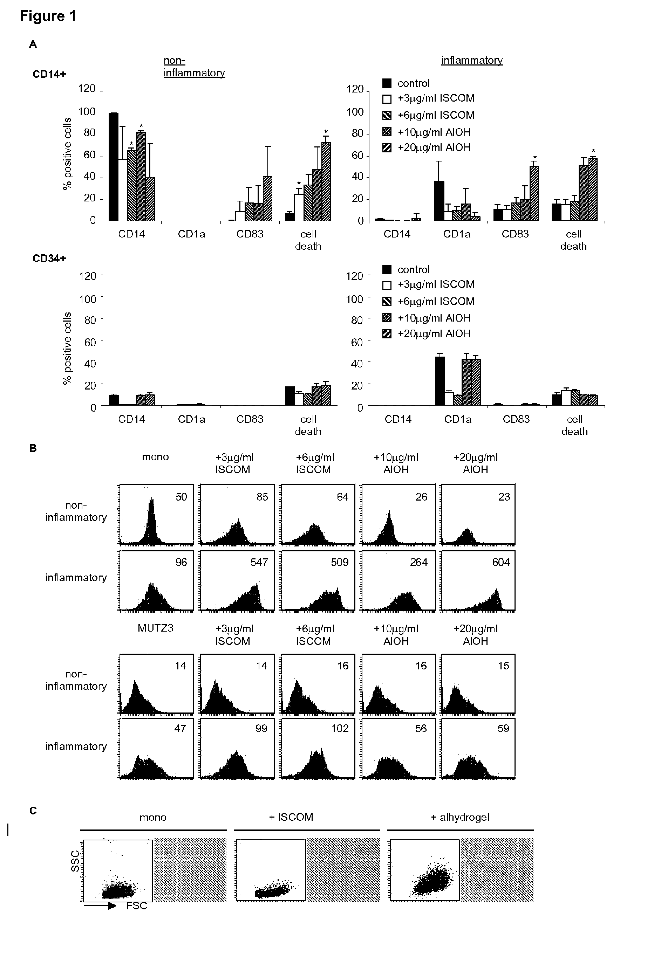

**Fig. 1****: Aluminum hydroxide (Alhydrogel**^{®}**) and Immune Stimulating Complexes (MATRIX M**^{®}**) induce monocyte activation, but no DC differentiation in vitro.**
   **A).** CD14⁺ monocytes (upper graphs) and CD34⁺ MUTZ3 progenitors (lower graphs) were cultured in the presence of 3 or 6 µg/ml Immune Stimulating Complexes (ISCOM) or 10 or 20 µg/ml aluminium hydroxide (AIOH) either under non-inflammatory conditions (left) or inflammatory conditions in the presence of GM-CSF and IL-4 (right). Shown are the average percentages of cells positive for CD14, CD1a, CD83 and propidium-iodide (dead cells) (n=3). P-values are given compared to the control cultures (no adjuvant); * *P* < 0.05.
   **B).** CD86 fluorescence histogram plots are shown from CD14⁺ monocytes (upper two rows) and CD34⁺ MUTZ3 cells (bottom two rows) under non-inflammatory and inflammatory conditions stimulated with the different concentrations of AIOH and ISCOM (representative of 3 experiments). Mean fluorescence intensities are shown in the top right corners of each plot.
   **C).** Forward/Side scatter (FSC/SSC) dot plots and cytospin preparations of cultured cells 5 days after adjuvant stimulation are shown.

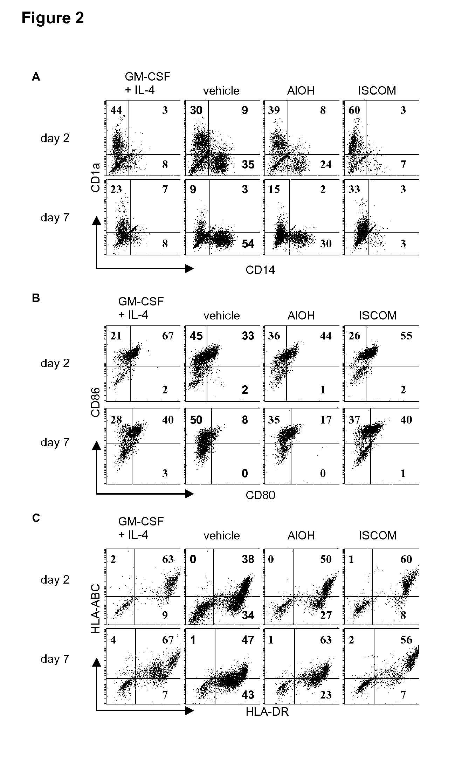

**Fig. 2****: Potent skin-DC activation by Immune Stimulating Complexes (Matrix M**^{®}**)**
   Human skin explants were injected with GM-CSF+IL4, vehicle (PBS), AIOH (1mg/ml) or Matrix M^{®} (ISCOM) (150 µg/ml) and migrated DC were harvested on day 2 and 7 and were phenotyped for CD14/CD1a **(A)** CD80/CD86 **(B)** and HLA-DR/HLA-ABC **(C)** expression. Percentages of cells positive for the indicated markers are given in the respective quadrants. Experiment representative of 5.
**Fig. 3****: Enhanced co-stimulatory molecule expression upon intradermal injection of Immune Stimulating Complexes (Matrix M**^{®}**).**
   Matrix M^{®} (ISCOM) significantly enhanced the percentage of cells double positive for CD80 and CD86 both at day 2 and 7. Furthermore, the total numbers of migrated skin-DC were enhanced upon i.d. Immune Stimulating Complex injection (data not shown).
**Fig. 4****: Adjuvant-mediated skin-DC activation corresponds with T cell stimulatory ability**
   **A).** Histogram plots of CFSE-labeled PBL stimulated with PBS-, GM+IL4-, ISCOM- or AIOH-activated skin-DC, showing CFSE dilution upon PBL division at day 6.
   **B).** Graph showing the average percentages of CFSE-diluted PBL stimulated with adjuvant-activated skin-DC through time on days 5, 6 and 7 (n=3).

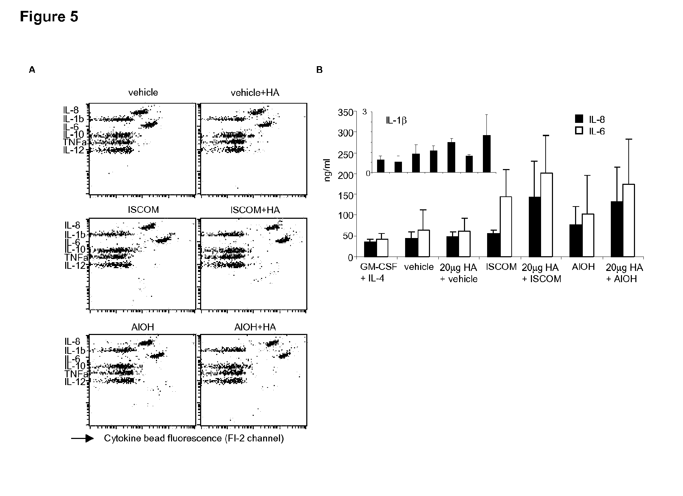

**Fig. 5****: Increased production of IL-6 and IL-8 by skin explants in response to HA antigen and adjuvants.**
   **A).** Typical CBA analysis dot plots showing differences in the cytokine profile secreted by skin explants i.d. injected with vehicle, MATRIX M^{®} (ISCOM) or aluminum hydroxide (AIOH) either or not in the presence of HA antigen (experiment representative of 3).
   **B).** Average secretion of IL-6 and IL-8 in skin explant-conditioned medium upon i.d. injection of the different adjuvants with or without HA antigen (n=3). Inset: average skin-explant secreted 1L-1β levels (ng/ml) under the same conditions as presented in the IL-6/IL-8 graph (n=3).

### Examples

### Adjuvants

MATRIX M^{®} was provided by ISCONOVA AB (Uppsala, Sweden) as two components; Matrix A (4 mg/ml saponin) and Matrix C (7.6 mg/ml saponin). For the experiments a 150 µg/ml mixture was made using 85% of Matrix A and 15% of Matrix C. Alhydrogel^{®} was purchased from Brenntag Biosector (Frederikssund, Denmark). The stock solution of Alhydrogel^{®} contained 2 % w/v (10 mg/ml) aluminum. Adjuvants were dissolved in PBS. The solutions containing 20 µg/ml HA (cell-derived H1N1; A/New Caledonia; batch 1037661 RW0603) were prepared by Solvay Biologicals (Weesp, The Netherlands).

### MUTZ3 cell line and interstitial DC cultures

The human CD34⁺ acute myeloid leukaemia cell line MUTZ-3 (DSMZ, Braunschweig, Germany) was cultured as follows: The cells were cultured in Minimum Essential Medium (MEM)-α (Lonza, Verviers, Belgium) supplemented with 20% fetal calf serum (FCS) (Perbio, Helsingborg, Sweden), 100IU/mi sodium penicillin (Yamanouchi Pharma, Leiderdorp, The Netherlands), 100µg/ml streptomycin (Radiumfarma-Fisopharma, Naples, Italy), 2.0mM L-glutamine (Invitrogen, Breda, The Netherlands) (pen/strep/glut), 0.05mM 2-mercapoethanol (2ME) (Merck, Darmstadt, Germany) and 10 % conditioned medium from the 5637-renal cell carcinoma cell line. Cells were cultured at 37°C and 5% CO₂ in 12 wells plates with concentrations between 0.2 x 10⁵ and 1.0 x 10⁶ and medium was refreshed every 3-4 days.
The MUTZ-3 derived interstitial DC (MUTZ3-DC) were cultured in 12 well tissue culture plates at a concentration of 1x10⁵/ml in the presence of 100IU/ml rhGM-CSF (Berlex, Seattle, USA), 10ng/ml interleukin-4 (IL-4) (R&D systems, Minnesota, USA) and 120U/ml TNF-α (Myltenyi Biotec B.V., Utrecht, The Netherlands) for 5-6 days. Interstitial DC (IDC) were also generated in vitro from human peripheral blood monocytes from healthy donors (MoDC) by adding 10ng/ml IL-4 and 10I1U/ml rhGM-CSF for 6 days.
Immature DC were matured by adding 2400U/ml TNFα, 100ng/ml IL-6 (R&D systems, Minnesota, USA), 25ng/ml IL-1β (Strathmann Biotec) and 1 µg/ml prostaglandin E2 (Sigma Aldrich) for 2 days. Immature and matured DC were phenotypically analyzed by flow cytometry. To test the effect of adjuvants on DC development and activation 10 µg/ml or 20 µg/ml aluminum hydroxide (AIOH), 3 µg/ml or 6 µg/ml Immune Stimulating Complexes (ISCOM) were added at the start of culture, either or not in the presence of DC-differentiating cytokines or after differentiation.

As can be seen in Fig. 1 A), rather than inducing DC activation, the model adjuvants Alhydrogel^{®} and MATRIX M^{®} induced cell death and monocyte activation in the in vitro cell-based assays.
Furthermore, in vitro stimulation of MoDC (mono) with MATRIX M^{®} only marginally increases the CD86 expression levels, but did not induce the maturation marker CD83.

### The skin explant model

Human skin specimens were obtained after informed consent from patients undergoing corrective breast or abdominal plastic surgery, following hospital guidelines. Skin explant cultures and skin DC migration assays, with subsequent analysis, were performed as described previously. Briefly, skin was i.d. injected with 20µl volume of PBS (negative control), 100IU/ml of rhGM-CSF and 20ng/ml of IL-4 (positive control), or with adjuvants (1mg/ml Alhydrogel^{®} (AIOH) or 150µg/ml MATRIX M^{®} (ISCOM)) with or without 20µg H1N1 HA antigen. 6 mm punch biopsies (10-20 samples/condition) were cultured, floating freely on IMDM containing 5% human pooled serum (HPS) (Sanquin, Amsterdam, the Netherlands), with their epidermal side up, before their removal 2 days later. The explants were discarded, and the medium, containing migrated cells, was harvested and pooled per test condition either on day 2 or day 7. Supernatants of the skin explant-conditioned medium were stored at -20°C for cytokine analysis. Absolute numbers of migrated DC were counted using trypan blue exclusion and flow-count fluorospheres (Beckman Coukter, Fullerton, CA).

### Antibodies and flow cytometry

PE- or FITC-labeled antibodies directed against human CD14, CD54, HLA-DR, HLA-ABC, CD1a, CD80, CD86 and DC-SIGN (BD Biosciences, Mountain view, CA), CD83, CD40, and Langerin (Immunotech, Marseille, France) were used for flow cytometric analysis. Antibody staining was performed in PBS supplemented with 0,1% bovine serum albumin (BSA) and 0,02% sodium-azide (NaN3) for 30 minutes at 4°C. Stained cells were analyzed on a FACS Calibur (BD Biosciences) using Cell Quest Software. To exclude dead cells in flow cytrometric analysis, 0,5µg/ml propidium iodide (ICN Biomedicals, Zoetermeer, The Netherlands) was added to each tube.

### Cytokine analysis

Cytokine content of skin explant-conditioned medium was analyzed using the cytokine bead array (Human Inflammation Kit, BD Biosciences, USA) for the simultaneous flow cytometric detection of IL-8, IL-1β, IL-6, IL-10, TNF-α, and IL-12p70, according to the manufacturer's instructions and using cytokine bead array analysis software (BD Biosciences).

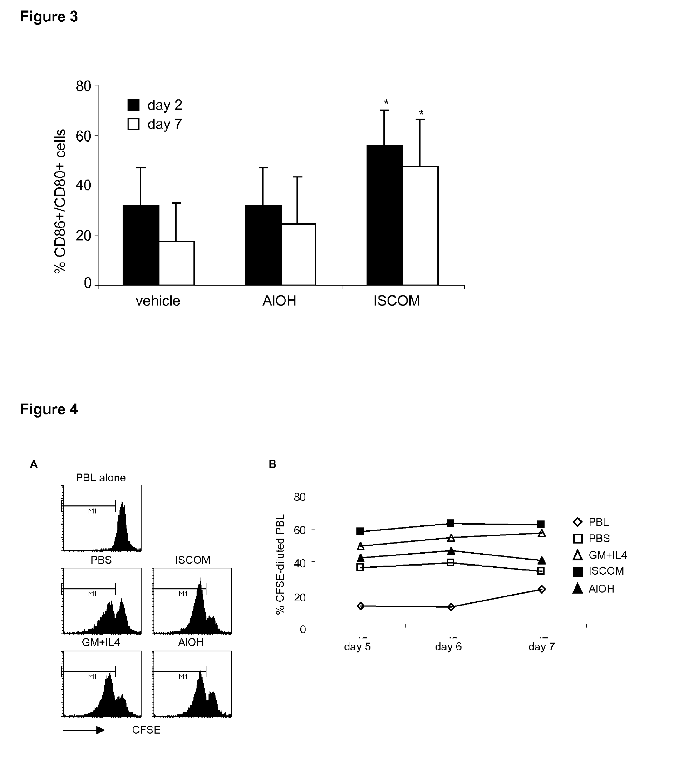

As can be seen in Fig. 2, upon GM-CSF+IL-4 (positive control) or ISCOM injection, DC received a strong activation signal and remained mature with high expression levels of co-stimulatory and antigen-presenting molecules and few CD14⁺ MΦ-like cells for at least 7 days (see also Fig. 3).

Furthermore, it is shown in Fig. 3 that ISCOM significantly enhanced the percentage of cells double positive for CD80 and CD86 both at day 2 and 7.
This ex vivo results obtained with the human ex vivo skin model are in strong contrast to the in vitro results shown in Fig. 1, where the stimulation of MoDC with ISCOM only marginally increases the CD86 expression levels. This indicates that the effect of the adjuvants observed in the in vitro model does not correlate with the ex vivo results.

Upon adjuvant injection, DC as well as surrounding tissue cells can respond by secreting various cytokines. Skin-tissue conditioned medium was harvested upon removal of the skin biopsies two days after adjuvant injection and media were analyzed for the presence of various inflammatory cytokines by means of a cytokine bead array. As shown in Figure 5A and B, ISCOM and AIOH injection mainly resulted in increased levels of IL-8 and IL-6. Interestingly, the highest levels of these cytokines were produced when HA antigen was given in combination with the adjuvants (Fig. 5A and 5B). IL-1β levels were somewhat increased when AIOH was injected in the presence of HA antigen (see inset Fig. 5B). Hardly any shifts in IL-10, TNF-α or IL-12p70 were observed (Fig. 5A).

### CFSE-Mixed Leukocyte Reaction (MLR)

Skin-DC induced peripheral blood leukocytes (PBL) proliferation was assessed by carboxyfluorescein succinimidyl ester (CFSE)-MLR. PBL were labeled with 1µM CFSE in PBS supplemented with 5% FCS for 7 minutes at 37°C (water bath). Cells were washed three times with PBS/5% FCS and one time with IMDM/5% HPS + pen/strep/glut. 0.1 Million PBL were stimulated with 10.000 skin-DC (10:1 ratio responder:stimulator cells). PBL proliferation, measured by CFSE-dilution, was analyzed in time by flow cytometry.

Along with improved DC migration and activation, i.d. injection of Immune Stimulating Complexes (ISCOM) resulted in DC with enhanced T cell stimulatory capacity (Fig. 4A and 4B). This corresponded with the high levels of co-stimulatory molecules still present 7 days after injection (Fig. 2 and Fig. 3). Figure 4A shows typical histogram plots, showing the most pronounced CFSE-dilution (as a measure of T cell proliferation) when ISCOM-activated DC were used as stimulator cells. The average percentage of PBL with diluted CFSE upon stimulation with day 7 skin-DC stimulated by PBS, GM+IL4, ISCOM or AIOH injection are depicted in Figure 4B (n=3). Cell proliferation was determined on different days of co-culture.

### Statistical analysis

Statistical analysis of the data was performed using the paired two-tailed T-test. Differences were considered statistically significant when p<0.05.

## Claims

1. A method for testing a candidate exogenous adjuvant in an ex vivo skin model, comprising the following steps:
a) providing an ex vivo skin tissue,
b) applying said exogenous adjuvant to be tested to the skin tissue, and
c) determining the immunological status of cells from the skin tissue.

2. The method according to claim 1, wherein in a step d) the immunological status of the cells from the skin tissue is compared to the immunological status of cells from a skin tissue to which no exogenous adjuvant has been applied or to which a reference adjuvant has been applied.

3. The method according to claim 2, wherein a exogenous adjuvant is selected as immunostimulative, if a cell fraction of at least about 30 %, preferably of at least about 40 %, more preferably of at least about 45 % or of at least about 50 %, and most preferably of at least about 60 % has the analyzed activation and/or maturation marker/s expressed.

4. The method according to any of the preceding items, wherein the method is used for testing a exogenous adjuvant for vaccine preparations.

5. The method according to any of the preceding claims, wherein the skin tissue is cultivated in culture medium for a time period of up to 7 days, preferably up to 5 days, more preferably up to 4 days, even more preferably up to 3 days, and most preferably up to 2 days.

6. The method according to any of claims 2 to 5, wherein in step d) the exogenous reference adjuvant is an adjuvant comprising at least one component selected from the group consisting of cholesterol, saponin, and phospholipid, optionally combined with amphipathic proteins.

7. The method according to any of the preceding claims, wherein the exogenous adjuvant is applied together with an antigen.

8. The method according to any of the preceding claims, wherein the exogenous adjuvant is applied together with a vaccine preparation to be tested.

9. The method according to any of the preceding claims, wherein the candidate exogenous adjuvant is
a) included in the vaccine preparation, or
b) added before or after the addition of the vaccine preparation.

10. The method according to any of the preceding claims, wherein the vaccine preparation contains as at least one antigen, preferably an inactivated or attenuated virus, more preferably a viral antigen such as a split virus antigen, a subunit virus antigen or a virosome, or the antigen comprises at least one component of a virus or a virus particle, preferably the antigen is an influenza virus particle or an avian influenza virus particle.

11. The method according to any of the preceding claims, wherein the vaccine preparation is an influenza vaccine preparation, preferably a pandemic or an epidemic influenza vaccine preparation, such as an avian influenza vaccine preparation or a seasonal influenza vaccine preparation.

12. The method according to any of the preceding claims, wherein the skin tissue is a mammalian skin tissue, preferably a human skin tissue.

13. The method according to any of the preceding claims, wherein in step 1 c) the immunological status of the cells from the skin tissue is determined at one time point or at different time points after the removal of the skin tissue from the culture medium.

14. The method according to claim 13, wherein the cells whose status is determined are dendritic cells, preferably dermal dendritic cells and monocyte-derived dendritic cells, Langerhans cells, and/or macrophage-like cells.

15. Use of the method according to any of the preceding claims for the development of, or as a prior step of selecting an exogenous adjuvant for the manufacture of, a vaccine preparation, preferably of an influenza vaccine preparation.

16. Use of an exogenous adjuvant selected by the testing method according to any of claims 1 to 14 as an exogenous adjuvant for a vaccine preparation, preferably for an influenza vaccine preparation, more preferably for a pandemic or an epidemic influenza vaccine preparation according to any of claims 10 or 11.

17. Use of an exogenous adjuvant according to claims 15 or 16, wherein the vaccine preparation contains an antigen and wherein the selected exogenous adjuvant displays an increased activating ability, more specifically a dendritic cell activating and maturating ability.

18. Use of an exogenous adjuvant according to any of claims 15 to 17, wherein the exogenous adjuvant is selected such that said exogenous adjuvant mainly induces a T_{H}1 or T_{H}2 and/or T_{H}17 cell subset, preferably a T_{H}1 or T_{H}2 subset, more preferably both subsets.
